# EUROPÄISCHE PATENTSCHRIFT

(11) **EP 1 261 390 B1**
(45) Veröffentlichungstag und Bekanntmachung des Hinweises auf die Patenterteilung: **04.08.2004**
(21) Anmeldenummer: 01909418.4
(22) Anmeldetag: 04.01.2001
(51) Int. Cl.: A61M 25/01

(54) **VORRICHTUNG ZUM EINFÜHREN EINES KATHETERS IN DIE HARNRÖHRE**
METHOD FOR INTRODUCING A CATHETER INTO THE URETHRA
DISPOSITIF POUR INTRODUIRE UN CATHETER DANS L'URETRE

(30) Priorität: 05.01.2000 DE 10000267; 24.03.2000 DE 10014857
(43) Veröffentlichungstag der Anmeldung: 04.12.2002
(73) Patentinhaber: Sauer, Manfred, 74931 Lobbach (DE)
(72) Erfinder: Sauer, Manfred, 74931 Lobbach (DE)
(74) Vertreter: Naumann, Ulrich, Dr.-Ing.
(86) Internationale Anmeldenummer: PCT/DE2001/000004
(87) Internationale Veröffentlichungsnummer: WO 2001/049354

(56) Entgegenhaltungen:
- EP-A- 0 916 305
- US-A- 3 332 424
- US-A- 3 683 928
- US-A- 5 417 666

## Beschreibung

Die Erfindung betrifft eine Vorrichtung zum Einführen eines Katheters in die Harnröhre, vorzugsweise zur Katheterisierung männlicher Personen, insbesondere bei Querschnittslähmung, mit einer Führungseinrichtung zum Einführen des Katheters.

Zur Bewältigung der Inkontinenz männlicher Personen, insbesondere bei Querschnittslähmung, wird u.a. der sogenannte intermittierende Selbstkatheterismus (ISK) angewandt, wonach sich die inkontinente männliche Person vier bis sechs Mal am Tag selbst katheterisiert. Um zwischen den Katheterisierungsphasen kontinent zu bleiben, wird üblicherweise vom behandelnden Arzt ein Medikament verordnet, welches die Blase reaktiviert bzw. ruhig stellt.

Bislang bekannte Blasenkatheter zur Ableitung von Urin sind jedoch in der Praxis problematisch, da nämlich beim Einführen des Katheters außen an der Eichelspitze oder im vorderen Bereich der Harnröhre angesiedelte Bakterien nach innen in die Harnröhre verschleppt werden. Mit den bislang bekannten Kathetern ist es jedenfalls auch bei bester Hygiene kaum möglich, den Katheter keimfrei durch die keimbelastete Zone bis in die Blase zu führen.

Zur Vermeidung einer zusätzlichen Keimbelastung der Harnröhre bzw. der Blase durch den Vorgang des Katheterisierens hat man bislang hohe Dosen desinfizierenden Gleitmittels für den Katheter verwendet, wobei die Nebenwirkungen des Gleitmittels und der desinfizierenden Zugaben bislang noch wenig erforscht sind. Zur ansatzweisen Vermeidung des Verschleppens von Keimen in der stark von Keimen besiedelten Harnröhrenöffnung wurde bereits ein ca. 15 mm langer Stutzen in die Harnröhrenöffnung eingeführt, durch den dann der eigentlich Katheter in die Hamröhre eingeschoben wird. Auch diese Vorgehensweise schließt in der Praxis nicht zuverlässig aus, dass auch über diesen Stutzen Keime in die Harnröhre weitergeschoben werden, die dann mit Hilfe des Katheters weiter durch die Harnröhre in die Blase gelangen.

Vor der eigentlichen Katheterisierung wird grundsätzlich empfohlen, den Eichelkopf beispielsweise mit Pflaumentupfern, zu desinfizieren. Während ein Patient im Verlaufe seines klinischen Aufenthalts hinreichend Zeit für eine solche desinfizierende Maßnahme hat, erscheint der dazu erforderliche Zeitaufwand nach dem Klinikaufenthalt nicht mehr akzeptabel. Folglich wird das empfohlene Maß an Hygiene und die insoweit erforderliche Desinfizierung vom Betroffenen reduziert und eine Sprühdesinfektion der Eichelspitze vorgenommen, wobei auch hier die erforderliche Einwirkzeit - meist aus Zeitmangel - unterschritten wird.

Letztendlich ist insbesondere bei der Selbstkatheterisierung stets davon auszugehen, dass die Eichelspitze und die Harnröhrenöffnung am stärksten keimbelastet sind und dass mit den bisherigen Systemen immer wieder die Gefahr besteht, dass dort angesiedelte Keime durch die Harnröhre in die Blase eingeschleppt werden. Entzündungen bzw. Infektionen sind die Folge.

Der vorliegenden Erfindung liegt daher die Aufgabe zugrunde, eine Vorrichtung zur Katheterisierung männlicher Personen anzugeben, wonach die Katheterisierung unter bestmöglichen hygienischen Bedingungen zeitsparend möglich ist.

Die zuvor genannte Aufgabe ist durch eine Vorrichtung mit den Merkmalen des Patentanspruches 1 gelöst. Danach ist eine gattungsbildende Vorrichtung siehe z.B. US-A-5 417 666 dadurch gekennzeichnet, dass die Führungseinrichtung einen zum Einschieben in den Hamröhreneingang bestimmten äußeren Trichter und einen durch den äußeren Trichter in die Harnröhre einschiebbaren inneren Trichter umfaßt, wobei der Katheter durch den inneren Trichter hindurch in die Harnröhre eingeführt wird.

Erfindungsgemäß ist erkannt worden, dass der als Führungseinrichtung zu bezeichnende, im Stand der Technik bislang bereits verwendete Stutzen nicht ausreicht, um ein Einschleppen von Keimen durch die Harnröhre in die Blase zu vermeiden. Des Weiteren ist erkannt worden, dass man dem im Stand der Technik auftretende Problem dadurch entgegentreten kann, dass man die Führungseinrichtung quasi teleskopartig ausgestaltet, nämlich durch die Kombination eines zum Einschieben in den Harnröhreneingang bestimmten äußeren Trichters mit einem durch den äußeren Trichter weiter in die Harnröhre einschiebbaren inneren Trichter. Beide Trichter bilden eine teleskopartig arbeitende Einrichtung, wobei der äußere Trichter zunächst wie der im Stand der Technik bekannten Stutzen arbeitet und der innere Trichter in diesen äußeren Trichter eingeschoben und mit seinem vorderen Ende durch den äußeren Trichter hindurch in die Harnröhre gelangt. Erst danach wird der Katheter - ohne Berührung der Eichelspitze, der Harnröhre oder des äußeren Trichters - durch den inneren Trichter hindurch in die Harnröhre eingeführt, so dass der Katheter mit keimbelasteten Bereichen gar nicht erst in Berührung kommt. Folglich wird erfindungsgemäß erreicht, dass die Führungseinrichtung eine teleskopartige Schleuse bildet, so dass das Verschleppen von Keimen zur Blase hin weitestgehend vermieden ist. Außerdem gewährleistet die erfindungsgemäße Vorrichtung mit der dort vorgesehenen Führungseinrichtung eine einfache und dabei sichere Handhabung, zumal die Führungseinrichtung nicht nur zum Vermeiden des Einschleppens von Keimen dient, sondern auch eine Art Zwangsführung für den eigentlichen Katheter vorgibt. Insoweit ist die Verletzungsgefahr beim Selbstkatheterismus ganz erheblich reduziert.

Im Rahmen einer ganz besonders vorteilhaften Ausgestaltung weist der äußere Trichter einen das Einschieben in die Harnröhre begrenzenden und im eingeschobenen Zustand den Harnröhreneingang bzw. die Eichelspitze abdeckenden Rand auf. Dieser Rand deckt den Hamröhreneingang schirmartig ab, so dass nach dem Einschieben des äußeren Trichters der mit Bakterien bzw. Keimen besiedelte Bereich unter Vermeidung einer weiterreichenden Kontaktierung abgedeckt ist. Dazu kann der Rand in seiner Form zumindest weitgehend der Eichelspitze bzw. der Eichel angepasst sein.

In weiter vorteilhafter Weise ist der innere Trichter ähnlich ausgebildet, weist nämlich ebenfalls einen das Einschieben in die Harnröhre begrenzenden und im eingeschobenen Zustand am Rand des äußeren Trichters auf dessen Innenseite zur Anlage kommenden Rand auf. Dabei ist der innere Trichter länger als der äußere Trichter ausbildet, so dass sich der innere Trichter mit seinem freien Ende durch den äußeren Trichter hindurch in die Harnröhre einschieben läßt. Beispielsweise könnte der äußere Trichter eine Länge von 15 mm und der innere Trichter eine Länge von 40 mm aufweisen, so dass sich der innere Trichter ohne eine unmittelbare Berührung der Eichelspitze oder des Harnröhreneingangs in die Harnröhre einschieben läßt. Erst nach vollständig eingeschobenem innerem Trichter wird dann der Katheter durch den inneren Trichter hindurch in die Harnröhre eingeführt.

Hinsichtlich der konkreten Ausgestaltung des inneren Trichters sei ergänzend erwähnt, dass der Rand des inneren Trichters in etwa der Form der Innenseite des äußeren Trichters angepasst sein kann, so dass ein weitestmögliches Einschieben des inneren Trichters bis hin zur gegenseitigen Anlage der beiden Ränder möglich ist, aber ein Durchrutschen vermieden wird.

Nun sollten die beiden Trichter im sterilisierten bzw. keimfreien Zustand möglichst auch mit Gleitmittel versehen sein. Dazu könnte der äußere Trichter an seinem zum Einschieben in den Hamröhreneinang dienenden freien Ende geschlossen und beim Durchschieben des inneren Trichters öffenbar sein. Ebenso könnte der innere Trichter an seinem zum Einschieben in den äußeren Trichter dienenden freien Ende zunächst geschlossen und beim Einschieben des Katheters öffenbar sein.

Der die beiden Trichter schließende Verschluss könnte derart gestaltet sein, dass die freien Enden der Trichter sollbruchstellenähnlich, vorzugsweise kreuzförmig, geschlitzt sind. Im Konkreten könnten die freien Ende der Trichter knospenartig ausgebildet sein, wobei beim Auftreten eines inneren Widerstandes ein Öffnen der Trichter nach außen hin erfolgt.

Bereits zuvor ist erwähnt worden, dass der Verschluss der beiden Trichter nicht nur zur sterilen Bereitstellung dient, sondern auch die Bildung eines Reservoirs innerhalb der Trichter ermöglicht, in dem Gleitmittel lagert. So könnte der vordere Bereich des äußeren Trichters wie auch der vordere Bereich des inneren Trichters als Reservoir für ein Gleitmittel dienen, wodurch sich ein ganz besonders vorteilhafter Effekt dadurch ergibt, dass beim Durchschieben des inneren Trichters die Außenwandung des inneren Trichters mit Gleitmittel benetzt wird und dann erst mit der Harnröhre in Berührung kommt. Ähnlich verhält es sich beim anschließenden Einschieben des Katheters, der nämlich durch das Gleitmittel-Reservoir des inneren Trichters hindurchgeschoben wird, sich mit Gleitmittel benetzt und so in die Harnröhre gelangt.

Des Weiteren ist wesentlich, dass der äußere Trichter äußerlich mit einem Gleitmittel benetzt sein kann, so dass sich auch dieser unproblematisch in die Öffnung der Harnröhre einschieben läßt.

Bei dem Gleitmittel kann es sich um handelsübliche, zum Katheterisieren empfohlene Gleitmittel, so beispielsweise um Öl, Vaseline oder dergleichen handeln. Dem Gleitmittel ist in ganz besonders vorteilhafter Weise ein Desinfektionsmittel beigemengt, so dass auch durch diese Maßnahme das Einschleppen von Keimen weitestgehend vermieden ist. Dem Gleitmittel könnte ebenso ein Medikament oder ein Narkotikum beigemengt sein, und zwar je nach Bedarf und Indikation.

Die Trichter sind in vorteilhafter Weise aus einem vorzugsweise weichen Kunststoff gefertigt, wobei sich die Verwendung von Silikon ganz besonders eignet. Dabei können die Trichter als Spritzgußteile ausgeführt sein, wobei der innere Trichter zumindest geringfügig fester bzw. härter als der äußere Trichter ausgeführt sein sollte.

Bereits zuvor ist die Funktionsweise der Führungseinrichtung erörtert worden. Zur einfachen Handhabung könnte die Vorrichtung im konfektionierten Zustand der Führungseinrichtung angeboten werden, wobei dabei der innere Trichter bereits in den äußeren Trichter zumindest geringfügig eingesteckt ist. Beide Trichter sind dabei an ihren freien Enden geschlossen, so dass in den sich dabei ergebenden Reservoirs Gleitmittel bevorratet wird. Des Weiteren ist der äußere Trichter an seiner Außenwandung ebenfalls mit Gleitmittel benetzt, so dass die gesamte Führungseinrichtung ohne das Erfordernis eines weiteren Gleitmittels und ohne das Erfordernis weiterreichender Hilfsmittel gehandhabt werden kann.

Zum Erhalt des sterilen Zustandes ist der innere Trichter an seinem einlaßseitigen Ende zunächst geschlossen, wobei der äußere Trichter wiederum durch den inneren Trichter zumindest weitestgehend geschlossen ist. Der Verschluss des inneren Trichters könnte als abziehbare Folie, als Stopfen oder dergleichen ausgeführt sein. Darüberhinaus könnte der Verschluss mit einem zur Handhabung und zur Öffnung dienenden Bügel oder mit einer entsprechenden Schnur, einem Band oder dergleichen, ausgestattet sein, so dass auch insoweit die Handhabung vereinfacht ist.

Die konfektionierte Führungseinrichtung kann darüberhinaus in einem Behältnis steril bereitgestellt werden. Dieses Behältnis könnte zumindest weitgehend der Form der gesamten Führungseinrichtung, d. h. der beiden Trichter, angepasst sein. Auch das Behältnis könnte wiederum ein Reservoir für Gleitmittel umfassen, wobei der äußere Trichter mit seinem zum Einführen in den Harnleiter dienenden freien Ende bzw. Bereich in dem Reservoir lagert, so dass die Außenwandung des äußeren Trichters stets mit Gleitmittel, Desinfektionsmittel und/oder einer medikamentösen Zugabe benetzt ist.

Das Behältnis zur Aufnahme der Führungseinrichtung könnte aus Kunststoff gefertigt sein, so dass hier insbesondere ein Kunststoff-Tiefziehteil als Behältnis verwendbar ist. Dabei könnte das Behältnis mit einer abziehbaren Folie oder mit einem Stopfen bzw. Deckel verschlossen sein, so dass die gesamte Führungseinrichtung stets in einem geschlossenen Behältnis - steril - dem Anwender zur Verfügung gestellt wird. Die Handhabung ergibt sich dabei äußerst einfach dahingehend, dass das Behältnis geöffnet, die bereits ineinandersteckenden Trichter entnommen und der äußere Trichter in den Hamröhreneingang bis zur Anlage des Randes eingeführt wird. Da der äußere Bereich zumindest im unteren Bereich äußerlich mit einem desinfizierenden Gleitmittel beschichtet ist, ist bereits insoweit das Einschieben von Keimen reduziert. Nachdem der äußere Trichter in den Harnröhreneingang eingesetzt ist, wird der innere Trichter durch das Gleitmittel-Reservoir hindurch in die Harnröhre nachgeschoben. Auch insoweit ist das weiterreichende Einschieben bzw. verschleppen von Keimen verhindert, zumal auch dieses Gleitmittel mit Desinfektionsmittel versehen sein kann. Schließlich wird die Öffnung des inneren Trichters durch Abziehen der Folie oder dergleichen geöffnet, so dass bei Anlage des Randes des inneren Trichters auf dem Rand des äußeren Trichters nunmehr der Katheter eingeführt werden kann, wobei bei diesem Einführvorgang sich der Katheter automatisch mit dem im unteren Bereich des inneren Trichters gelagerten Gleitmittel benetzt, so dass auch insoweit ein sicheres Einführen und eine Vermeidung des Einschleppens von Keimen realisiert ist. Die weitere Katheterisierung erfolgt wie gewohnt.

Es gibt nun verschiedene Möglichkeiten, die Lehre der vorliegenden Erfindung in vorteilhafter Weise auszugestalten und weiterzubilden. Dazu ist einerseits auf die nachgeordneten Ansprüche, andererseits auf die nachfolgende Erläuterung eines

Ausführungsbeispiels der Erfindung anhand der Zeichnung zu verweisen. In Verbindung mit der Erläuterung des bevorzugten Ausführungsbeispiels der Erfindung anhand der Zeichnung werden auch im allgemeinen bevorzugte Ausgestaltungen und Weiterbildungen der Lehre erläutert. In der Zeichnung zeigt:
- Fig. 1: in einer schematischen Seitenansicht ein Ausführungsbeispiel einer erfindungsgemäßen Vorrichtung, wobei dort die Teile der Führungseinrichtung separiert sind,
- Fig. 2: in einer schematischen Seitenansicht den Gegenstand aus Fig. 1 im vorkonfektionierten Zustand, d. h. mit ineinandergesteckten Trichtern,
- Fig. 3: in einer schematischen Seitenansicht den Gegenstand aus den Fig. 1 und 2 mit völlig eingeschobenem inneren Trichter und mit angedeutetem Katheter,
- Fig. 4: in einer schematischen Seiten- und Unteransicht die jeweils freien, geschlossenen Enden der Trichter mit Sollbruchstellen und
- Fig. 5: in einer schematischen Seitenansicht ein Behältnis zur Aufnahme der Vorrichtung aus den Fig.1 bis 4.

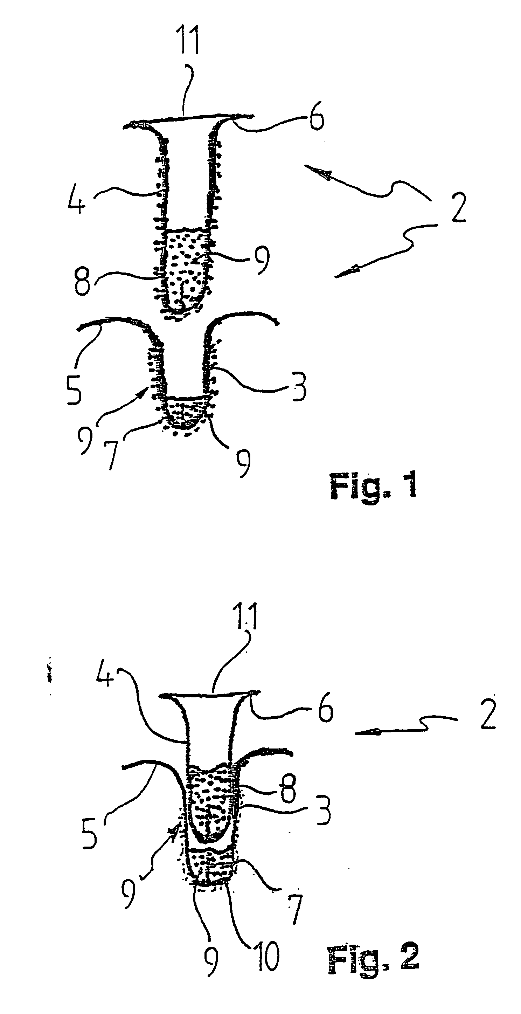

Die Fig. 1 bis 4 zeigen ein Ausführungsbeispiel einer erfindungsgemäßen Vorrichtung zum Einführen eines schlauchartigen Katheters 1 in die Harnröhre, nämlich zur Katheterisierung männlicher Personen, wobei es sich dabei insbesondere um die Anwendung bei Querschnittslähmungen handelt. Die Vorrichtung umfasst eine Führungseinrichtung 2 zum Einführen des Katheters 1 in die Harnröhre.

Erfindungsgemäß weist die Führungseinrichtung 2 einen zum Einschieben in den Hamröhreneingang bestimmten äußeren Trichter 3 und einen durch den äußeren Trichter 3 in die Harnröhre einschiebbaren inneren Trichter 4 auf. Der Katheter 1 wird durch den inneren Trichter 4 hindurch in die Harnröhre geschoben.

Die Fig. 1 bis 3 zeigen deutlich, dass der äußere Trichter 3 einen das Einschieben in die Harnröhre begrenzenden und im eingeschobenen Zustand den Harnröhreneingang bzw. die Eichelspitze abdeckenden Rand 5 aufweist. Dieser Rand 5 ist in seiner Form der Eichelspitze bzw. der Eichel angepasst.

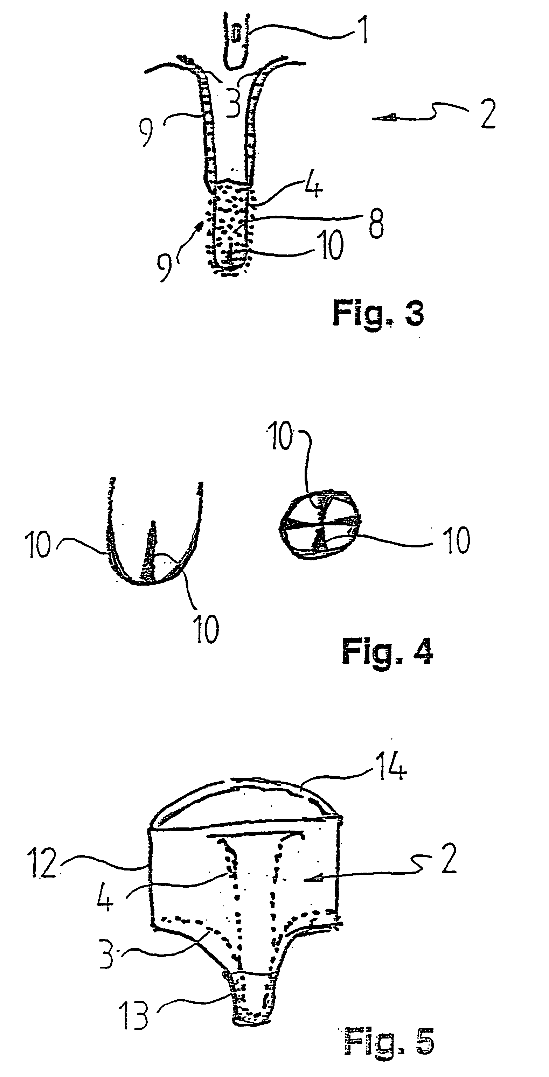

Ähnlich verhält es sich mit dem inneren Trichter 4, der einen das Einschieben in die Harnröhre begrenzenden und im eingeschobenen Zustand am Rand 5 des äußeren Trichters 3, auf dessen Innenseite zur Anlage kommenden Rand 6 aufweist. Fig. 3 zeigt dabei besonders deutlich, dass der Rand 6 des inneren Trichters 4 in etwa der Form der Innenseite des Randes 5 des äußeren Trichters 3 angepasst ist.

Die Fig. 1 bis 3 unter Hinzuziehung von Fig. 4 zeigen besonders deutlich, dass sowohl der äußere Trichter 3 wie auch der innere Trichter 4 am jeweils freien Ende geschlossen und beim Durchschieben des inneren Trichters 4 bzw. beim Einschieben des Katheters 1 öffenbar sind. Insoweit bilden die freien bzw. unteren Enden der Trichter 3, 4 Reservoirs 7, 8 zur Aufnahme eines Gleitmittels 9, wobei dieses Gleitmittel 9 wahlweise mit einem Desinfektionsmittel bzw. Narkotikum versetzt ist.

Fig. 4 zeigt deutlich, dass die freien Enden der Trichter 3, 4 kreuzförmig geschlitzt sind, wodurch sich eine knospenartige Ausbildung ergibt. Die dort vorgesehenen Sollbruchstellen 10 ermöglichen ein Öffnen des jeweiligen Trichters 3, 4 beim Einschieben des inneren Trichters 4 bzw. beim Durchschieben des Katheters 1. Dabei wird das Gleitmittel 9 gegebenenfalls nebst Desinfektionsmittel freigesetzt, so dass der durchgeschobene innere Trichter 4 und der eingeschobene Katheter 1 an der Außenseite jeweils hinreichend mit Gleitmittel 9 benetzt ist.

In den Fig.1 bis 3 ist des Weiteren angedeutet, dass der äußere Trichter 3 an seiner Außenwandung ebenfalls mit Gleitmittel 9 benetzt ist, um nämlich das Einschieben bzw. Einführen des äußeren Trichters 3 unter Vermeidung eines Mitschleppens von Bakterien zu ermöglichen.

Die beiden Trichter 3, 4 sind - bei dem hier gewählten Ausführungsbeispiel - aus Silikon gefertigt, wobei es sich dabei im Konkreten um Spritzgußteile handeln kann. Der innere Trichter 4 ist dabei zumindest geringfügig fester bzw. härter als der äußere Trichter 3 ausführt, wodurch sich die Handhabung ebenfalls bei einer Reduktion der Verletzungsgefahr vereinfacht. An dieser Stelle sei angemerkt, dass zur Herstellung der beiden Trichter 3, 4 beliebige geeignete Materialien und entsprechende Herstellverfahren in Frage kommen.

Fig. 2 zeigt die Führungseinrichtung 2 im konfektionierten Zustand, wonach nämlich der innere Trichter 4 in den äußeren Trichter 3 geringfügig eingesteckt ist. Die beiden Reservoirs 7, 8 sind mit Gleitmittel 9 hinreichend gefüllt, wobei der äußere Trichter 3 an seiner Außenwandung ebenfalls mit Gleitmittel 9 versehen ist.

Des Weiteren läßt Fig. 2 erkennen, dass der innere Trichter 4 an seinem einlaßseitigen Ende geschlossen ist, nämlich eine dort abziehbare Folie 11 aufweist. Alternativ dazu könnte auch ein Deckel oder eins sonstiger Verschluss - wie auch immer gestaltet - vorgesehen sein. Bei dem hier gewählten Ausführungsbeispiel ist die Folie 11 am äußeren Rand 6 des inneren Trichters 4 adhäsiv befestigt und läßt sich durch einfaches Abziehen entfernen. Bei der Darstellung in Fig. 3 ist der innere Trichter 4 bereits vollständig in den äußeren Trichter 3 eingeschoben. Die Folie 11 ist entfernt und der Katheter 1 ist dort kurz vor dessen Einführung angedeutet.

Fig. 5 zeigt andeutungsweise, dass die konfektionierte Führungseinrichtung 2 in einem Behältnis 12 steril bereitgestellt wird. Dieses Behältnis 12 ist der Form der Führungseinrichtung 2 angepasst. Der äußere Trichter 3 ist mit seinem zum Einführen dienenden freien Ende bzw. mit seinem unteren Bereich in einem dort vorgesehenen Reservoir 13 gelagert, wobei dieses Reservoir 13 ebenfalls Gleitmittel 9 beinhaltet. Eine hinreichende Versorgung der Außenwandung des äußeren Trichters 3 mit Gleitmittel 9 ist damit gewährleistet.

Das Behältnis 12 ist aus Kunststoff gefertigt, wobei es sich bei dem hier gewählten Ausführungsbeispiel um ein Kunststoff-Tiefziehteil handelt. Zur keimfreien Lagerung ist das Behältnis 12 mit einer abziehbaren Folie 14 geschlossen, wobei anstelle der Folie 14 auch ein Deckel, ein Stopfenoder dergleichen vorgesehen sein kann.

Die erfindungsgemäße Vorrichtung hat den Vorteil der vereinfachten Lagerhaltung und Logistik in der Klinik, im Fachhandel sowie im privaten Haushalt. Eine Art Baukastenlösung ergibt sich insbesondere dadurch, dass unterschiedlich große Führungseinrichtungen und Katheter mit unterschiedlichen Durchmessern frei kombinierbar sind. Letztendlich ist neben den herkömmlichen Kathetern und sonstigen Desinfektionsmitteln lediglich die im Sinne einer Teleskopschleuse dienende Vorrichtung - keimfrei abgepackt - zur Verfügung zu stellen.

Hinsichtlich der Funktionsweise sowie hinsichtlich einer weiterreichenden Beschreibung wird zur Vermeidung von Wiederholungen auf die Beschreibungseinleitung verwiesen.

Abschließend sei ganz besonders darauf hingewiesen, dass das zuvor erörterte Ausführungsbeispiel lediglich zur Erörterung der beanspruchten Lehre dient, diese jedoch nicht auf das Ausführungsbeispiel einschränkt.

## Patentansprüche

1. Vorrichtung zum Einführen eines Katheters (1) in die Harnröhre, vorzugsweise zur Katheterisierung männlicher Personen, insbesondere bei Querschnittslähmung, mit einer Führungseinrichtung (2) zum Einführen des Katheters (1),
**dadurch gekennzeichnet, dass** die Führungseinrichtung (2) einen zum Einschieben in den Harnröhreneingang bestimmten äußeren Trichter (3) und einen durch den äußeren Trichter (3) in die Harnröhre einschiebbaren inneren Trichter (4) umfasst, wobei der Katheter (1) durch den inneren Trichter (4) hindurch in die Hamröhre einführbar ist.

2. Vorrichtung nach Anspruch 1, **dadurch gekennzeichnet, dass** der äußere Trichter (3) einen das Einschieben in die Harnröhre begrenzenden und im eingeschobenen Zustand den Harnröhreneingang bzw. die Eichelspitze abdeckenden Rand (5) aufweist, wobei der Rand (5) in seiner Form der Eichelspitze bzw. der Eichel angepasst sein kann.

3. Vorrichtung nach Anspruch 1 oder 2, **dadurch gekennzeichnet, dass** der innere Trichter (4) einen das Einschieben in die Harnröhre begrenzenden und im eingeschobenen Zustand am Rand (5) des äußeren Trichters, auf dessen Innenseite zur Anlage kommenden Rand (6) aufweist, wobei der Rand (6) des inneren Trichters in etwa der Form der Innenseite des äußeren Trichters (3) angepasst sein kann.

4. Vorrichtung nach einem der Ansprüche 1 bis 3, **dadurch gekennzeichnet, dass** der äußere Trichter (3) an seinem zum Einschieben in den Harnröhreneingang dienenden freien Ende geschlossen und beim Durchschieben des inneren Trichters (4) öffenbar ist und/oder dass der innere Trichter (4) an seinem zum Einschieben in den äußeren Trichter (3) dienenden freien Ende geschlossen und beim Einschieben des Katheters (1) öffenbar ist, wobei die freien Enden der Trichter (3, 4) sollbruchstellenähnlich, vorzugsweise kreuzförmig, geschlitzt oder knospenartig ausgebildet sind.

5. Vorrichtung nach Anspruch 4, **dadurch gekennzeichnet, dass** der vordere Bereich des äußeren Trichters (3) als Reservoir (7) für ein Gleitmittel (9) dient und/oder dass der vordere Bereich des inneren Trichters (4) als Reservoir (8) für ein Gleitmittel (9) dient.

6. Vorrichtung nach einem der Ansprüche 1 bis 5, **dadurch gekennzeichnet, dass** der äußere Trichter (3) äußerlich mit einem Gleitmittel (9) benetzt ist.

7. Vorrichtung nach Anspruch 5 oder 6, **dadurch gekennzeichnet, dass** es sich bei dem Gleitmittel (9) um Öl, oder Vaseline handelt und/oder dass dem Gleitmittel (9) ein Desinfektionsmittel und/oder ein Medikament und/oder ein Narkotikum beigemengt ist.

8. Vorrichtung nach einem der Ansprüche 1 bis 7, **dadurch gekennzeichnet, dass** die Trichter (3, 4) aus einem vorzugsweise weichen Kunststoff, vorzugsweise aus Silikon gefertigt sind.

9. Vorrichtung nach einem der Ansprüche 1 bis 8, **dadurch gekennzeichnet, dass** der äußere Trichter (3) zumindest geringfügig weicher als der innere Trichter (4) ausgeführt ist.

10. Vorrichtung nach einem der Ansprüche 1 bis 9, **dadurch gekennzeichnet, dass** im konfektionierten Zustand der Führungseinrichtung (2) der innere Trichter (4) in den äußeren Trichter (3) zumindest geringfügig eingesteckt ist.

11. Vorrichtung nach einem der Ansprüche 1 bis 10, **dadurch gekennzeichnet, dass** der innere Trichter (4) am einlaßseitigen Ende geschlossen ist, wobei der Verschluss als abziehbare Folie (11) oder als Deckel oder als Stopfen ausgeführt sein kann.

12. Vorrichtung nach Anspruch 11, **dadurch gekennzeichnet, dass** der Verschluss mit einer zur Handhabung und zum Öffnen dienenden Schlaufe oder einem entsprechenden Bügel oder mit einer zur Handhabung und zum Öffnen dienenden Schnur ausgestattet ist.

13. Vorrichtung nach einem der Ansprüche 1 bis 12, **dadurch gekennzeichnet, dass** die konfektionierte Führungseinrichtung (2) in einem Behältnis (12) steril bereitgestellt wird, wobei das Behältnis (12) zumindest weitestgehend der Form der Führungseinrichtung (2) angepasst sein kann.

14. Vorrichtung nach Anspruch 13, **dadurch gekennzeichnet, dass** das Behältnis (12) ein Reservoir (13) für Gleitmittel (9) umfasst und dass der äußere Trichter (3) mit seinem zum Einführen dienenden freien Ende bzw. Bereich in dem Reservoir (13) lagert, wobei das Behältnis (12) mit einer abziehbaren Folie (14) oder mit einem Deckel oder einem Stopfen verschlossen sein kann, wobei der Verschluß zur einfachen Handhabung des Öffnens mit integriertem Bügel oder einer entsprechenden Schlaufe ausgestaltet sein kann und wobei das äußere Behältnis (12) als Verschluss einen Faltmechanismus aufweisen kann, der vorzugsweise mittels Lasche oder durch seitlichen Druck öffenbar ist.

## Claims

1. Device for introducing a catheter (1) into the urethra, preferably for catheterising male persons, in particular in the case of paraplegia, having a guide device (2) for introducing the catheter (1),
**characterised in that** the guide device (2) comprises an outer funnel (3), which is intended for insertion into the urethra orifice, and an inner funnel (4), which can be inserted through the outer funnel (3) into the urethra, the catheter (1) being able to be introduced through the inner funnel (4) into the urethra.

2. Device according to claim 1, **characterised in that** the outer funnel (3) has a rim (5) which limits the insertion into the urethra and which covers the urethra orifice or the tip of the glans penis in the inserted state, the rim (5) being able to be adapted in terms of its shape to the tip of the glans penis or the glans penis.

3. Device according to claim 1 or 2, **characterised in that** the inner funnel (4) has a rim (6) which limits the insertion into the urethra and which, in the inserted state, comes to rest against the rim (5) of the outer funnel at the inner side thereof, the rim (6) of the inner funnel being able to be adapted approximately to the shape of the inner side of the outer funnel (3).

4. Device according to any one of claims 1 to 3, **characterised in that** the outer funnel (3) is closed at the free end thereof, which is used for insertion into the urethra orifice, and can be opened when the inner funnel (4) is inserted, and/or **in that** the inner funnel (4) is closed at the free end thereof, which is used for the insertion into the outer funnel (3), and can be opened when the catheter (1) is inserted, the free ends of the funnels (3, 4) being constructed in a manner similar to desired breaking points, preferably in a cross-like, slotted or bud-like manner.

5. Device according to claim 4, **characterised in that** the front region of the outer funnel (3) acts as a reservoir (7) for a lubricant (9) and/or **in that** the front region of the inner funnel (4) acts as a reservoir (8) for a lubricant (9).

6. Device according to any one of claims 1 to 5, **characterised in that** the outer funnel (3) is wetted externally with a lubricant (9).

7. Device according to claim 5 or 6, **characterised in that** the lubricant (9) is oil or Vaseline and/or **in that** a disinfectant and/or a medicament and/or a narcotic is added to the lubricant (9).

8. Device according to any one of claims 1 to 7, **characterised in that** the funnels (3, 4) are produced from a preferably soft plastics material, preferably from silicone.

9. Device according to any one of claims 1 to 8, **characterised in that** the outer funnel (3) is constructed so as to be at least slightly softer than the inner funnel (4).

10. Device according to any one of claims 1 to 9, **characterised in that**, in the assembled state of the guide device (2), the inner funnel (4) is fitted at least to a small extent into the outer funnel (3).

11. Device according to any one of claims 1 to 10, **characterised in that** the inner funnel (4) is closed at the end located at the inlet side, the closure being able to be constructed as a removable film (11) or a cover or a plug.

12. Device according to claim 11, **characterised in that** the closure is provided with a loop, which is used for handling and opening, or a corresponding curved member or a cord which is used for handling and opening.

13. Device according to any one of claims 1 to 12, **characterised in that** the assembled guide device (2) is provided in a sterile state in a container (12), the container (12) being able to be adapted at least to the greatest possible extent to the shape of the guide device (2).

14. Device according to claim 13, **characterised in that** the container (12) comprises a reservoir (13) for lubricant (9) and **in that** the outer funnel (3) is positioned in the reservoir (13) by means of the free end or region thereof which is used for introduction, the container (12) being able to be closed by a removable film (14) or a cover or a plug, the closure being able to be constructed so as to have an integrated curved member or a corresponding loop for simple handling of the opening operation, and the outer container (12) being able to have a folding mechanism as the closure means, which can preferably be opened by means of a flap or by lateral pressure.

## Revendications

1. Dispositif pour l'introduction d'un cathéter (1) dans l'urètre, de préférence pour le cathétérisme de personnes humaines, en particulier dans le cas d'hémiplégie transversale, avec un dispositif de guidage (2) pour l'introduction du cathéter (1),
**caractérisé par le fait que** le dispositif de guidage (2) comprend un entonnoir (3) extérieur défini pour le coulissement dans l'entrée de l'urètre et un entonnoir (4) intérieur pouvant être introduit par coulissement à travers l'entonnoir (3) extérieur, le cathéter (1) pouvant être introduit à travers l'entonnoir (4) intérieur à travers l'urètre.

2. Dispositif selon la revendication 1,
**caractérisé par le fait que** l'entonnoir (3) extérieur présente un bord (5) limitant l'introduction dans l'urètre et, à l'état introduit, recouvrant l'entrée de l'urètre ou respectivement la pointe du gland, le bord (5) pouvant être adapté dans sa forme à la pointe du gland ou respectivement au gland.

3. Dispositif selon la revendication 1 ou 2,
**caractérisé par le fait que** l'entonnoir (4) intérieur présente un bord (6) limitant l'introduction dans l'urètre et, à l'état introduit, venant en appui sur le bord (5) de l'entonnoir (3) extérieur, sur sa face intérieure,le bord (6) de l'entonnoir intérieur pouvant être adapté approximativement à la forme de l'entonnoir (3) extérieur.

4. Dispositif selon l'une des revendications 1 à 3,
**caractérisé par le fait que** l'entonnoir (3) extérieur est fermé à son extrémité libre servant à l'introduction dans l'entrée de l'urètre et peut être ouvert lors de la traversée de l'entonnoir (4) intérieur et/ou que l'entonnoir (4) intérieur est fermé à son extrémité libre servant à l'introduction dans l'entonnoir (3) extérieur et peut être ouvert lors de l'introduction du cathéter (1), les extrémités libres des entonnoirs (3,4) étant conformées de manière analogue à une amorce de rupture, de préférence en forme de croix, fendus ou en forme de bourgeon.

5. Dispositif selon la revendication 4,
**caractérisé par le fait que** la zone antérieure de l'entonnoir (3) extérieur sert de réservoir (7) pour un lubrifiant (9) et/ou que la zone antérieure de l'entonnoir (4) intérieur sert de réservoir (8) pour un lubrifiant (9).

6. Dispositif selon l'une des revendications 1 à 5,
**caractérisé par le fait que** l'entonnoir (3) extérieur est humecté extérieurement par un lubrifiant (9).

7. Dispositif selon la revendication 5 ou 6,
**caractérisé par le fait qu'**il s'agit pour le lubrifiant (9) d'huile ou de vaseline et/ou qu'un agent de désinfection et/ou un médicament et/ou un narcotique sont mélangés au lubrifiant (9).

8. Dispositif selon l'une des revendications 1 à 7,
**caractérisé par le fait que** les entonnoirs (3,4) sont fabriqués en une matière synthétique de préférence molle, de préférence en silicone.

9. Dispositif selon l'une des revendications 1 à 8,
**caractérisé par le fait que** l'entonnoir (3) extérieur est réalisé au moins légèrement plus mou que l'entonnoir (4) intérieur.

10. Dispositif selon l'une des revendications 1 à 9,
**caractérisé par le fait que**, à l'état confectionné du dispositif de guidage (2) l'entonnoir (4) intérieur est enfoncé au moins légèrement dans l'entonnoir (3) extérieur.

11. Dispositif selon l'une des revendications 1 à 10,
**caractérisé par le fait que** l'entonnoir (4) intérieur est fermé à l'extrémité du côté d'entrée, la fermeture pouvant être réalisée sous forme d'une feuille (11) pelable ou sous forme de couvercle ou sous forme de bouchon.

12. Dispositif selon la revendication 11,
**caractérisé par le fait que** la fermeture est équipée d'un crochet fermé servant à la manipulation et à l'ouverture ou d'une barrette correspondante ou d'un cordon servant à la manipulation et à l'ouverture.

13. Dispositif selon l'une des revendications 1 à 12,
**caractérisé par le fait que** le dispositif de guidage (2) confectionné est mis à disposition stérile dans un récipient (12), le récipient (12) pouvant être adapté au moins largement à la forme du dispositif de guidage (2).

14. Dispositif selon la revendication 13,
**caractérisé par le fait que** le récipient (12) comprend un réservoir (13) pour un lubrifiant (9) et que l'entonnoir (3) extérieur est disposé, par son extrémité ou respectivement sa zone libre servant à l'introduction, dans le réservoir (13), le récipient (12) pouvant être fermé par une feuille (14) pelable ou par un couvercle ou un bouchon, la fermeture pouvant être équipée, pour une manipulation simple de l'ouverture, d'une barrette intégrée ou d'un crochet fermé correspondant et le récipient (12) extérieur pouvant présenter un mécanisme pliant en tant que fermeture, lequel peut être ouvert de préférence au moyen d'un collier ou par pression latérale.
